# EUROPEAN PATENT APPLICATION

(11) **EP 4 636 076 A1**
(43) Date of publication of application: **22.10.2025**
(21) Application number: 23903711.2
(22) Date of filing: 16.10.2023
(51) Int. Cl.: C12N 5/00

(54) **ORGANOID SORTER**

(30) Priority: 14.12.2022 KR 20220174402; 12.10.2023 KR 20230135653
(71) Applicant: Radahaim Co., Ltd., Seongnam-si, Gyeonggi-do 13466 (KR)
(72) Inventor: LEE, Heon Ju, Yongin-si Gyeonggi-do 17009 (KR); AN, Jee Young, Seoul 03066 (KR); LIM, Hyun Wook, Seoul 04726 (KR)
(74) Representative: Hautier IP - MC/EP
(86) International application number: PCT/KR2023/015961
(87) International publication number: WO 2024/128507

(57) **Abstract**

Organoid sorter of the present disclosure includes: an injection port into which a fluid containing organoids is injected, the organoids being spaced apart from each other to be injected one by one; a microfluidic channel connected to the injection port, includes a spiral region extending in a spiral shape, and generates a centrifugal force on the organoids while the organoids move along with the fluid; a plurality of collection chambers which are connected downstream of the microfluidic channel and connected so that the organoids are sorted by size and collected; a plurality of guide channels which connect each of the plurality of collection chambers and the microfluidic channel and guide the organoids so that the organoids are sorted by set size and introduced into each of the collection chambers; and outlets which are formed in each of the plurality of collection chambers and serve as recovery paths for the organoids.

## Description

### [Technical Field]

The present disclosure relates to an organoid sorter that sorts cultured organoids by size.

### [Background Art]

In the process of developing new drugs, animal testing on animals similar to humans is required before conducting clinical trials. Animals such as rats and rabbits are used for animal testing, but the ethics of this are problematic. In addition, since animal testing models differ from humans in genetic and biological characteristics, side effects that were not found in animal testing are found in humans, and drug testing using existing animal testing models has limitations in the reliability of drug test responses.

Moreover, as the US FDA recently removed the mandatory requirement of animal testing as a condition for new drug approval, various methods for new preclinical testing models for new drug development are being studied.

However, conventional 2D cell line culture methods have the disadvantage of being difficult to reproduce the unique properties and characteristics of tissues, and although cancer patient-derived xenograft models are utilized as an alternative, they have the disadvantage of being unsuitable for large-scale drug screening.

Therefore, organoids have recently attracted significant attention as a new preclinical testing model for new drug development.

Organoids are organoids that are three-dimensionally cultured or recombined from stem cells. They are derived from various organs in the body and contain stem cells inside, forming aggregates that can differentiate into cell bodies. In addition, organoids derived from each organ in the body have a structure very similar to that organ, so they can be used as models that can replace cell and animal experiments.

However, conventionally cultured organoids have had limitations in their use in actual drug development due to problems with consistency and reproducibility of research results. This is because cell culture varies depending on the laboratory environment, and even when cultured under the same conditions, the size and degree of differentiation vary depending on the batch, making it difficult to obtain statistically significant data.

To solve these conventional problems, 3D organoid culture and organoid sorting technologies are required.

Three-dimensional culture of organoids is a technology for culturing organoids by physically contacting the bottom surface of a medium, or the like, and it is expected that a large number of organoids can be cultured under uniform conditions. However, due to the characteristics of stem cells and life sciences, even when culture is performed under uniform conditions without physical contact with the bottom surface, only organoids with relatively homogeneous characteristics can be obtained, and organoids of completely identical sizes cannot be obtained. Therefore, in order to obtain more homogeneous organoids, a technology for sorting cultured organoids by size is necessary.

### [Disclosure]

### [Technical Problem]

An object of the present disclosure is to provide an organoid sorter that sorts cultured organoids by size.

### [Technical Solution]

According to one aspect of the present disclosure, there is provided an organoid sorter including: an injection port into which a fluid containing organoids is injected, the organoids being spaced apart from each other to be injected one by one; a microfluidic channel which is connected to the injection port, includes a spiral region extending in a spiral shape, and generates a centrifugal force on the organoids while the organoids move along with the fluid; a plurality of collection chambers which are connected downstream of the microfluidic channel and connected so that the organoids are sorted by size and collected; a plurality of guide channels which connect each of the plurality of collection chambers and the microfluidic channel and guide the organoids so that the organoids are sorted by set size and introduced into each of the collection chambers; and outlets which are formed in each of the plurality of collection chambers and serve as recovery paths for the organoids.

According to one aspect of the present disclosure, the plurality of guide channels include each inlet connected to the microfluidic channel, the inlet of each of the plurality of guide channels has a size that matches an upper limit of the size of the organoid to be sorted, and the inlets are disposed sequentially along an extension path of the microfluidic channel, and an inlet with a relatively smaller size is disposed upstream of an inlet with a relatively larger size.

According to one aspect of the present disclosure, the inlets of the plurality of guide channels are formed on an outer inner wall of the microfluidic channel.

According to one aspect of the present disclosure, the microfluidic channel may include the spiral region and a straight region extending from an end portion of the spiral region, and the inlets may be formed in the straight region. In addition, some of the inlets may be formed at the end portion of the spiral region, and the remaining inlets may be formed from the end portion of the spiral region adjacent to the straight region to the straight region, as formed in the straight region.

According to one aspect of the present disclosure, the spiral region extends at least 360 degrees centered on the injection port.

According to one aspect of the present disclosure, the microfluidic channel has a square cross-section.

According to one aspect of the present disclosure, the inlet is formed as a rectangle having an inlet length defined along an extension path of the microfluidic channel and a width defined to intersect the extension path, and an upper limit of the size of the organoid introduced into the inlet is determined by the inlet length.

According to one aspect of the present disclosure, the straight region is extended while having a square cross-section of a constant size.

According to one aspect of the present disclosure, the organoid sorter has a magnet below the collection chamber facing the microfluidic channel.

According to one aspect of the present disclosure, the organoid sorter further includes a liquid channel connecting the end portion of the straight region of the microfluidic channel and the plurality of collection chambers to each other.

### [Advantageous Effects]

Using the organoid sorter according to the present disclosure, organoids sorted by set size can be obtained. This makes it possible to mass-produce homogeneous organoids.

Therefore, it is advantageous in ensuring consistency and reproducibility of research results since it enables providing a large number of organoid samples with homogeneous characteristics and sizes for new drug experiments, or the like.

### [Description of Drawings]

FIG. 1 is a flowchart of an organoid product method according to one embodiment of the present disclosure.
FIG. 2 is a plan view of an organoid sorter according to one embodiment of the present disclosure.
FIG. 3 is a perspective view of the organoid sorter according to one embodiment of the present disclosure.
FIG. 4 is a diagram illustrating an example of using the organoid sorter according to one embodiment of the present disclosure.
FIG. 5 is a diagram illustrating an organoid moving along a microfluidic channel in the organoid sorter according to one embodiment of the present disclosure.
FIG. 6 is a diagram illustrating an organoid being sorted by size and collected in a collection chamber in the organoid sorter according to one embodiment of the present disclosure.

### [Best Mode]

Hereinafter, an embodiment of the present disclosure will be described with reference to the attached drawings.

The present disclosure may have various modifications and various embodiments, and specific embodiments are illustrated in the drawings and described in detail in the detailed description. However, this is not intended to limit the present disclosure to specific embodiments, and it should be understood that it includes all modifications, equivalents, and substitutes included in the spirit and technical scope of the present disclosure. In explaining the present disclosure, if it is judged that a specific description of related known technology may obscure the gist of the present disclosure, the detailed description thereof will be omitted.

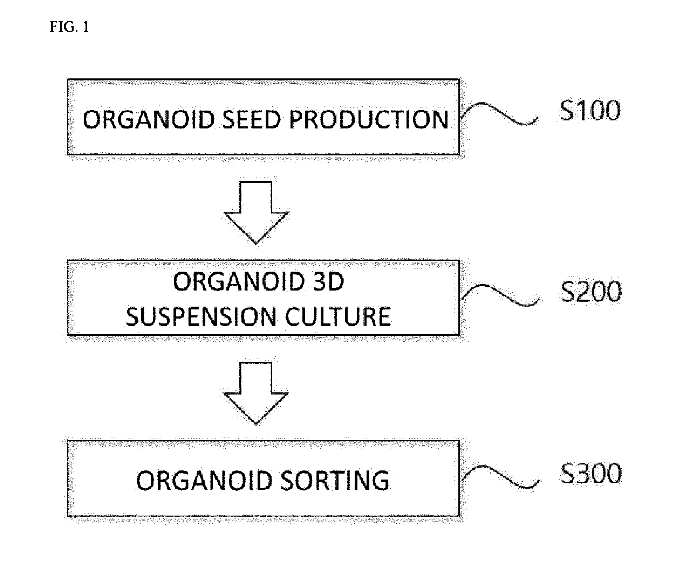

FIG. 1 is a flowchart of an organoid production method according to the present disclosure. Referring to FIG. 1, the organoid 3D culture method includes an organoid seed production step (S100), an organoid 3D suspension culture step (S200), and an organoid sorting step (S300).

According to an embodiment of the present disclosure, the production of the organoid seed (S100) may be achieved by forming organoid seed droplets suspended in a liquid medium within a microchannel and gelling the organoid seed droplets. The organoid seeds are produced in a spherical shape with a uniform size within the liquid medium.

The "organoid seed" refers to a seed formed using an organoid seed composition and subjected to seeding to be an organoid, and "composition for producing organoids" refers to a cell composition including stem cells provided for culturing the organoids.

According to an embodiment of the present disclosure, the organoid 3D suspension culture (S200) may be performed in a microfluidic chip having microchannels and an organoid 3D suspension culture device having a magnet array.

The microfluidic chip may be used, which includes: an injection port through which a fluid including organoid seeds and a culture medium are injected; a microchannel connected to the injection port and through which the organoid seeds are injected one body one at a set injection interval and moved and held at a set culture position and cultured; an outlet through which the culture medium and cultured organoids are discharged; and a valve capable of blocking the movement of fluids to the injection port and the outlet. The organoid seeds include magnetic nanoparticles.

The magnet array may be provided with magnets that are respectively arranged on opposite surfaces of the microfluidic chip and provide a magnetic force to suspend and hold the organoid seed in the microchannel at the culture position.

The 3D suspension culture of organoids (S200) includes steps of organoid seed injection, organoid seed suspension and holding, organoid culture, and organoid recovery, and may be performed to include an alignment step for aligning organoid seeds prior to organoid seed suspension and holding.

The organoid seed injection step includes injecting the organoid seed into the microchannel together with a fluid. The organoid seed includes magnetic nanoparticles. The fluid is not limited by any specific component and is intended to inject the organoid seed into the microchannel without damaging the organoid seed. The fluid may be a saline solution or a culture medium.

The organoid seeds are injected into the microchannel one by one in a state of being mixed in the fluid.

The organoid alignment step is a step in which the organoid seeds supplied one by one to the microchannel are uniformly arranged in a state of being spaced apart from each other along the microchannel. As the organoid seeds are arranged at a set position within the microchannel, combining the magnet array to the microfluidic chip is advantageous in that the organoid seeds are suspended and held at the set position by magnetic force without clumping, or the like. The organoid seeds are aligned using alignment grooves formed on the inner surface of the microchannel and gravity. To this end, the microfluidic chip may be rotated 90° from a horizontal position where the organoid seeds are introduced to a vertical position.

The organoid seed suspension and holding step involves attaching a magnetic array to both surfaces of the microfluidic chip so that the organoid seeds are suspended in a direction opposite to gravity within the culture medium by magnetic force. Accordingly, the organoid seeds are prevented from being pressed in a specific direction by gravity, thereby preventing growth in the direction of gravity from being hindered or inhibited, thereby enabling practical three-dimensional culture.

The organoid culture step is a step in which differentiation of organoids is induced and grown while replacing the culture medium. The nutrients necessary for organoid growth are supplied by periodically replacing the culture medium.

The organoid recovery step is the step of recovering the organoid from the microchannel. The magnet array is separated from the microfluidic chip so that the organoid seed can move along the fluid flow within the microchannel. The organoid is discharged through the outlet of the microfluidic chip together with the fluid and recovered.

In the organoid sorting step (S300), organoids generated by the 3D suspension culture step are sorted into groups according to size.

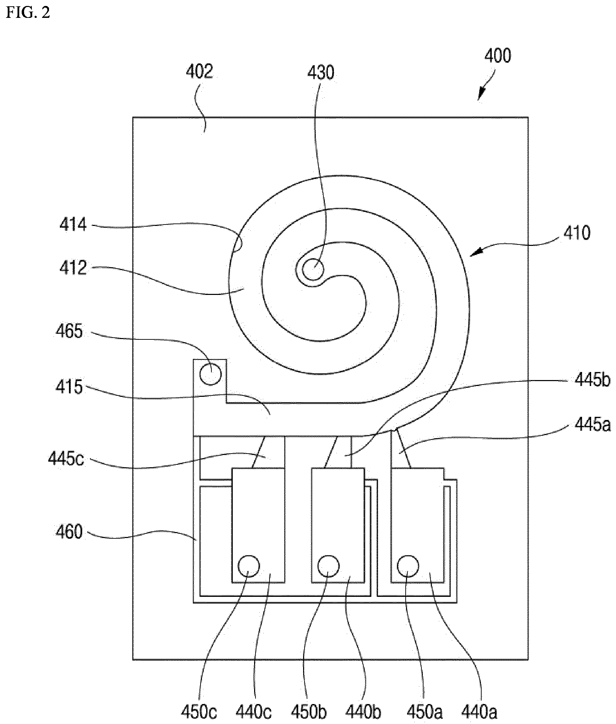

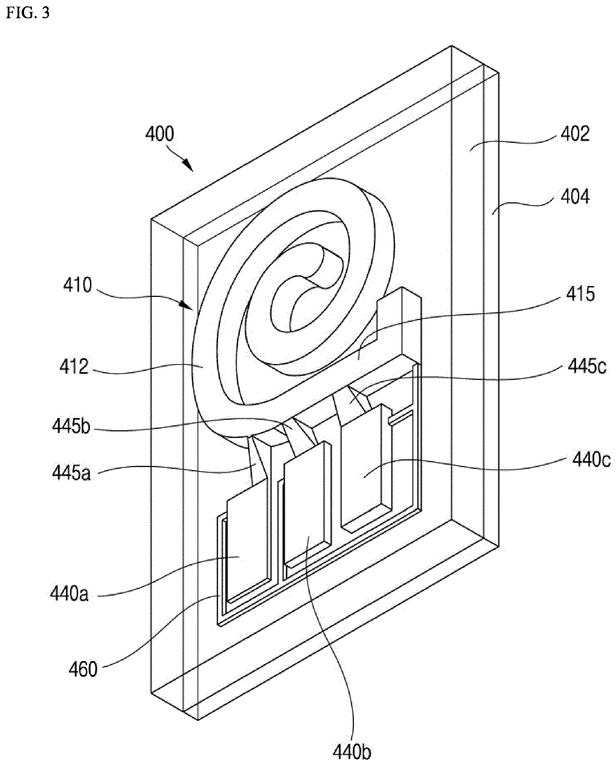

FIG. 2 is a plan view of an organoid sorter according to one embodiment of the present disclosure, and FIG. 3 is a perspective view of the organoid sorter according to one embodiment of the present disclosure.

An organoid sorter 400 is formed by joining a first plate 402 and a second plate 404.

Referring to the drawings, a microfluidic channel 410 and collection chambers 440a, 440b, and 440c are formed in the first plate 402 through a patterning process, a molding process, a processing process, or the like, and an injection port 430 and outlets 450a, 450b, and 450c are formed in a second plate 404. The first and second plates 402 and 404 manufactured in this manner are joined to each other to manufacture the organoid sorter 400 having the microfluidic channel 410 and the collection chambers 440a, 440b, and 440c formed inside. The formation locations of the microfluidic channel 410, the collection chambers 440a, 440b, and 440c, the injection port 430, the outlets 450a, 450b, and 450c, and the like are not limited by the matters illustrated in the drawing. For example, the injection port 430 may be formed in the first plate 402.

The first plate 402 and the second plate 404 may be manufactured from inorganic materials such as glass or silicon, and may be manufactured using polymer materials such as poly(dimethyl) siloxane (PDMS) or polymethylmethacrylate (PMMA).

According to an embodiment of the present disclosure, the organoid sorter 400 has the injection port 430 through which the fluid containing organoids is injected. Through the injection port 430, organoids cultured in an organoid culture device may be injected one by one in a state of being mixed in the fluid while being spaced apart from each other.

The organoid sorter 400 includes the microfluidic channel 410 formed starting from the injection port 430.

The microfluidic channel 410 includes a spiral region 412 extending in a spiral shape and a straight region 415 extending in a straight shape from an end portion of the spiral region 412. The microfluidic channel 410 is formed as a square channel having a square cross-section.

The spiral region 412 is a movement path of the organoid that generates centrifugal force in the organoid as the organoid injected with the fluid moves in a spiral direction. The spiral region 412 is formed to extend at least 360° with the injection port 430 as the center so that centrifugal force can be generated in the organoid. The organoid moves while being adhered to an outer inner wall 414 of the microfluidic channel 410 by the centrifugal force. The outer inner wall 414 means an inner wall located radially outward with respect to the center of the spiral. A force is applied radially outward to the outer inner wall 414 with respect to the center of the spiral.

In the specification of the present disclosure, the terms "upstream" and "downstream" are used to define the location of a specific portion of a microfluidic channel 410. The upstream and downstream relate to the flow of fluid injected into an injection port and flowing along the microfluidic channel 410. The fluid flows from upstream to downstream.

The straight region 415 is a stabilizing region for stabilizing the organoid that has moved while being attached to the outer inner wall 414 of the spiral region 412. As illustrated in FIG. 3, the microfluidic channel 410 may have a square cross-section, and the straight region 415 extends in a straight line while having a square cross-section of a constant size.

This prevents organoids moving from the spiral region 412 to the straight region 415 from experiencing drag reduction, pressure drop, or the like due to a sudden decrease in flow rate. The straight region 415 is connected to the end portion of the spiral region 412 that can amplify centrifugal force, thereby solving the problem of organoids being mixed due to a sudden decrease in flow rate or pressure increase.

The organoid sorter 400 includes the plurality of collection chambers 440a, 440b, and 440c connected to the straight region 415 near the end portion of a spiral region 412 on the downstream side of the microfluidic channel 410.

The collection chambers 440a, 440b, and 440c are chambers in which organoids are sorted and accommodated by size, and a collection chamber corresponding to organoids with relatively small sizes is disposed on the upstream side of the microfluidic channel.

In an embodiment of the present disclosure, organoids are separated into three sizes, and a first collection chamber 440a, a second collection chamber 440b, and a third collection chamber 440c are sequentially positioned from the upstream side to the downstream side. The size of organoids collected in the first collection chamber 440a is relatively smaller than that of organoids collected in the second collection chamber 440b, and the size of organoids collected in the second collection chamber 440b is smaller than that of organoids collected in the third collection chamber 440c.

Referring to the drawings, the first collection chamber 440a is connected through a first guide channel 445a near the end portion of the spiral region 412, and the second and third collection chambers 440b and 440c are connected to second and third guide channels 445b and 445c in the straight region 415.

The first to third collection chambers 440a, 440b, and 440c are located below the straight region 415 within the plate of the organoid sorter 400.

The microfluidic channel 410 and the plurality of collection chambers 440a, 440b, and 440c are connected through guide channels 445a, 445b, and 445c.

When the connection portion of the guide channels 445a, 445b, and 445c and the microfluidic channel 410 is defined as an inlet, the inlet includes an inlet IN-1 of the first guide channel 445a connecting the first collection chamber 440a and the microfluidic channel 410, an inlet IN-2 of the second guide channel 445b connecting the second collection chamber 440b and the microfluidic channel 410, and an inlet IN-3 of the third guide channel 445c connecting the third collection chamber 440c and the microfluidic channel 410.

The inlets of the guide channels 445a, 445b, and 445c are formed on the outer inner wall 414 of the microfluidic channel 410 and are formed as square slits. The inlets of the guide channels 445a, 445b, and 445c are arranged sequentially in size from the end portion of the spiral region 412 adjacent to the straight region 415 to the straight region 415.

Each inlet may be formed as a rectangle having an opening length defined along an extension path of the microfluidic channel 410 and a width defined in a direction intersecting the extension path, the width being identical to the width of the microfluidic channel 410.

According to the present disclosure, the upper limit of the size of the organoid introduced through the inlet is determined by the opening length of the inlet, and the opening length between each inlet satisfies the size relationship IN-3 > IN-2 > IN-1. That is, the size of the inlet is determined by the opening length of the inlet, and becomes a criterion in sorting of the organoid.

The organoid introduced by the size of inlet IN-1 is defined as a first size, small size organoid, the organoid introduced by the size of inlet IN-2 is defined as a second size, medium size organoid, and the organoid introduced by the size of inlet IN-3 is defined as a third size, large size organoid. The small size organoid is collected into the first collection chamber 440a through IN-1, the medium size organoid is collected into the second collection chamber 440b through IN-2, and the large size organoid is collected into the third collection chamber 440c through IN-3.

As described above, the size of the inlet serves as the criterion in the sorting of the organoids entering each of the collection chambers 440a, 440b, and 440c, and since the size of the inlet increases toward the downstream, larger organoids are collected in the collection chambers 440a, 440b, and 440c toward the downstream. The cross-section of each of the guide channels 445a, 445b, and 445c may be formed in a tapered shape that gradually decreases from the inlet, which is the connection portion with the microfluidic channel 410, to the connection portion with the collection chambers 440a, 440b, and 440c.

A liquid movement channel 460 is connected between the end portion of the microfluidic channel 410 and each of the collection chambers 440a, 440b, and 440c. The liquid movement channel 460 forms a flow path for liquids such as saline solution and culture medium, excluding organoids.

The liquid movement channel 460 provides a flow path for the fluid, that is, the transport solution, so that the transport solution can continuously flow. Accordingly, the transport of the organoid may be prevented from being interrupted by the stoppage of the transport solution. The transport solution is formed thinly so as to prevent the inflow of the organoid.

When the cultured organoids are injected one by one together with fluid through the injection port 430 using this configuration, the centrifugal force is applied to each organoid as the organoids are transported along the spiral region 412 of the microfluidic channel 410.

Due to centrifugal force, organoids with larger mass experience greater forces. Since the mass of an organoid depends on its size, a relatively larger force is applied to organoids with larger sizes.

The organoids move along the outer inner wall 414 of the microfluidic channel 410 under centrifugal force and progress from the spiral region 412 to the straight region 415. Depending on the progression path and the sizes of the inlets of the guide channels 445a, 445b, and 445c connecting the collection chambers 440a, 440b, and 440c and the microfluidic channel 410, organoids of a smaller size may be sequentially sorted and accommodated in the collection chamber on the upstream side.

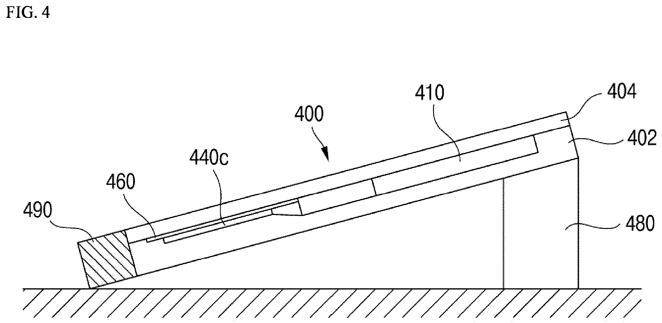

FIG. 4 is a usage state diagram of the organoid sorter according to one embodiment of the present disclosure.

Referring to FIG. 4, the organoid sorter 400 is used by slanting the first plate 402 in which the microfluidic channel 410 is formed, with the first plate 402 positioned downward, by a support member 480.

Accordingly, the organoids injected together with the transport liquid through the injection port 430 (see FIG. 2) move along the microfluidic channel 410 as centrifugal force is applied by the injection pressure and gravity. Since the gravity is applied, it is advantageous to set the injection pressure low to prevent damage to the organoids.

According to an embodiment of the present disclosure, the organoid sorter 400 may further include a magnet 490 below the collection chambers 440a, 440b, and 440c facing the microfluidic channel 410. When the organoid includes magnetic nanoparticles, an additional attractive force is applied by the magnet 490 to collect the organoid into the collection chambers 440a, 440b, and 440c.

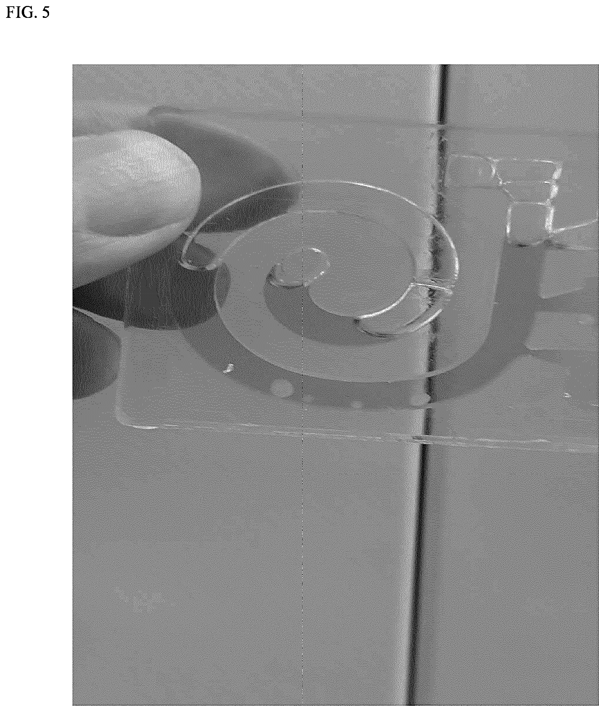

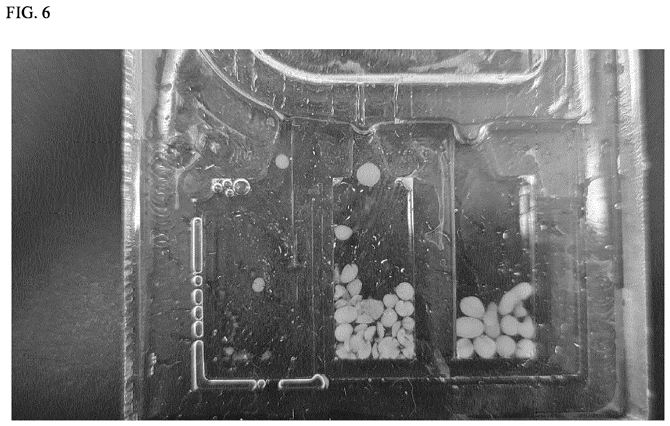

FIG. 5 is a diagram illustrating the organoid moving along the microfluidic channel in the organoid sorter according to one embodiment of the present disclosure, and FIG. 6 is a diagram illustrating the organoid being sorted by size and collected in a collection chamber in an organoid sorter according to one embodiment of the present disclosure.

The microchannel size was manufactured to be 5 mm × 5 mm, the inlet of the first guide channel connected to the first collection chamber was formed as a slit having an opening length of 1 mm, the inlet of the second guide channel connected to the second collection chamber was formed as a slit having an opening length of 2 mm, and the inlet of the third guide channel connected to the third collection chamber was formed as a slit having an opening length of 3 mm.

The organoid sorter was placed at a 15-degree angle using the support member, and the fluid containing the organoids was injected at 5 to 10 ml/min.

Referring to FIG. 5, it can be seen that organoids of different sizes move along the outer wall of the microchannel under the action of centrifugal force.

Referring to FIG. 6, it can be seen that organoids are collected into each collection chamber while being sorted by size.

## Claims

1. An organoid sorter comprising:
an injection port into which a fluid containing organoids is injected, the organoids being spaced apart from each other to be injected one by one;
a microfluidic channel which is connected to the injection port, includes a spiral region extending in a spiral shape, and generates a centrifugal force on the organoids while the organoids move along with the fluid;
a plurality of collection chambers which are connected downstream of the microfluidic channel and connected so that the organoids are sorted by size and collected;
a plurality of guide channels which connect each of the plurality of collection chambers and the microfluidic channel and guide the organoids so that the organoids are sorted by set size and introduced into each of the collection chambers; and
outlets which are formed in each of the plurality of collection chambers and serve as recovery paths for the organoids.

2. The organoid sorter of claim 1, wherein the plurality of guide channels include each inlet connected to the microfluidic channel,
the inlet of each of the plurality of guide channels has a size that matches an upper limit of the size of the organoid to be sorted, and
the inlets are disposed sequentially along an extension path of the microfluidic channel, and an inlet with a relatively smaller size is disposed upstream of an inlet with a relatively larger size.

3. The organoid sorter of claim 2, wherein the inlets of the plurality of guide channels are formed on an outer inner wall of the microfluidic channel.

4. The organoid sorter of claim 2, wherein the microfluidic channel includes the spiral region and a straight region extending from an end portion of the spiral region, and
at least some of the inlets are formed in the straight region.

5. The organoid sorter of claim 3, wherein the spiral region extends at least 360 degrees centered on the injection port.

6. The organoid sorter of claim 3, wherein the microfluidic channel has a square cross-section,
the inlet is formed as a rectangle having an inlet length defined along the extension path of the microfluidic channel and a width defined to intersect the extension path, and
the upper limit of the size of the organoid introduced into the inlet is determined by the inlet length.

7. The organoid sorter of claim 4, wherein the straight region is extended while having a square cross-section of a constant size.

8. The organoid sorter of claim 4, further comprising a liquid channel connecting an end portion of the straight region of the microfluidic channel and the plurality of collection chambers to each other.
